(19) Europäisches Patentamt
European Patent Office
Office européen des brevets

(11) EP 4 299 080 A1

(12) **DEMANDE DE BREVET EUROPEEN**

(43) Date de publication:
**03.01.2024 Bulletin 2024/01**

(21) Numéro de dépôt: **22305941.1**

(22) Date de dépôt: **28.06.2022**

(51) Classification Internationale des Brevets (IPC):
***A61K 49/04*** *(2006.01)* ***A61K 49/00*** *(2006.01)*
***A61K 49/18*** *(2006.01)* ***A61L 24/04*** *(2006.01)*
***C08F 220/28*** *(2006.01)* ***C08F 226/10*** *(2006.01)*
***A61K 9/00*** *(2006.01)* ***A61K 31/00*** *(2006.01)*
***A61K 38/00*** *(2006.01)*

(52) Classification Coopérative des Brevets (CPC):
**A61K 49/0442; A61K 31/00; A61K 38/00; A61K 49/0002; A61K 49/048; A61K 49/1818; A61L 24/043; C08F 220/286; C08F 226/10;**
A61K 9/0019; A61K 9/5026; A61K 9/5094

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(71) Demandeur: **Guerbet**
**93420 Villepinte (FR)**

(72) Inventeurs:
- **MOINE, Laurence**
  **92210 Saint Cloud (FR)**
- **FRANÇOIS, Baptiste**
  **75010 Paris (FR)**
- **FOUGERE, Olivier**
  **60128 Mortefontaine (FR)**
- **LOUGUET, Stéphanie**
  **33170 Gradignan (FR)**

(74) Mandataire: **Regimbeau**
**20, rue de Chazelles**
**75847 Paris Cedex 17 (FR)**

(54) **MONOMÈRE RADIO-OPAQUE ET MICROSPHÈRES D'EMBOLISATION LE COMPRENANT**

(57) La présente invention porte sur un composé de formule (A) suivante, notamment utile en tant que monomère radio-opaque :

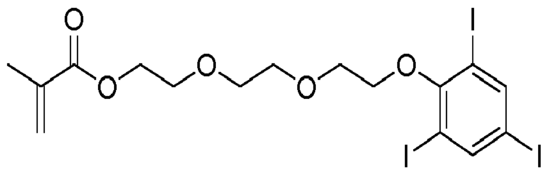

(A)

En outre, l'invention porte sur des microsphères d'embolisation radiopaques à base d'au moins :
- 20 % à 90 % de monomère hydrophile
- 5 % à 50 % de composé de formule (A)
- 1 % à 15 % de monomère réticulant hydrophile non biodégradable et
- 0,1 % à 10 % d'agent de transfert.

L'invention porte également sur une composition pharmaceutique comprenant au moins une micropshère d'embolisation selon l'invention, en association avec un véhicule pharmaceutiquement acceptable, avantageusement pour une administration par voie parentérale.

L'invention porte en outre sur un kit comprenant une composition pharmaceutique selon l'invention en association avec un véhicule pharmaceutiquement acceptable pour une administration par voie parentérale, et un moyen d'injection.

EP 4 299 080 A1

## Description

DOMAINE DE L'INVENTION

La présente invention concerne un nouveau monomère radio-opaque halogéné, notamment destiné à être utilisé au sein d'une matrice réticulée entrant dans la composition de microsphères d'embolisation.

ART ANTERIEUR

L'occlusion vasculaire thérapeutique (c'est-à-dire l'embolisation) est utilisée pour prévenir ou traiter certaines conditions pathologiques *in situ.* Elle peut être réalisée au moyen de cathéters permettant, sous contrôle d'imagerie, de positionner des agents d'occlusion particulaire (c'est-à-dire des emboles ou agents emboliques) dans le système circulatoire. Elle a une variété d'applications médicales telles que le traitement des malformations vasculaires, des processus hémorragiques, ou des tumeurs, y compris, par exemple, les fibromes utérins, les tumeurs primitives ou secondaires du foie. Par exemple, l'occlusion vasculaire peut provoquer une nécrose tumorale et éviter une opération plus invasive. Cette technique d'occlusion peut également être couplée à la fourniture d'un agent anticancéreux dans le cadre de la chimioembolisation. Cela permet d'augmenter la concentration locale tout en limitant l'exposition systémique de médicament par une injection ciblée, ainsi que son temps de résidence dans la tumeur. Dans le cas de malformations vasculaires, l'occlusion vasculaire permet de normaliser le flux sanguin vers les tissus normaux, d'aider à la chirurgie en limitant le risque d'hémorragie. Dans les processus hémorragiques, l'occlusion vasculaire peut entraîner une diminution du débit, ce qui favorise la cicatrisation de la plaie artérielle. En outre, selon les pathologies traitées, l'embolisation peut être utilisée à des fins temporaires ou à des fins permanentes.

Les agents d'embolisation sont classiquement introduits dans un vaisseau sanguin via un cathéter, en particulier un microcathéter, dont le diamètre est inférieur à celui du vaisseau à traiter. Les agents d'embolisation pour l'occlusion vasculaire comprennent par exemple les liquides d'embolisation (colles acryliques, gels), les dispositifs mécaniques, les particules et les microsphères d'embolisation polymériques. Le choix d'un matériau spécifique dépend de nombreux facteurs, tels que le type de lésion à traiter, le type de cathéter à utiliser et le besoin d'une embolisation temporaire ou permanente.

Les microsphères d'embolisation à base de polymères sont particulièrement utiles aux fins thérapeutiques précitées. Elles peuvent être biodégradables pour une embolisation temporaire, telles que décrites dans les demandes WO 2012/120139 et WO 2012/120138, ou non biodégradables pour une embolisation permanente.

Par exemple, le produit Embosphere® (Biosphere Medical) correspond à des microsphères non biodégradables à base de trisacryl (N-acryloyl-2-amino-2- hydroxymethylpropane-1,3 diol), et de gélatine. Des microsphères non biodégradables à base de copolymères acryliques et d'alcool polyvinylique (PVA) ont également été proposées pour l'embolisation permanente (Osuga et al. (2002) J. Vasc Interv Radiol. 13: 929-34).

En outre, afin d'être visibles en imagerie par rayons X, les microsphères d'embolisation peuvent être rendues radio-opaques par addition d'une entité ou d'un monomère radio-opaque dans leur composition. De telles microsphères d'embolisation radio-opaques sont décrites dans les demandes WO 2021 /069527 et WO 2021 /069528. Les microsphères de ces demandes intègrent un monomère radio-opaque dénommé MAOETIB, de formule suivante :

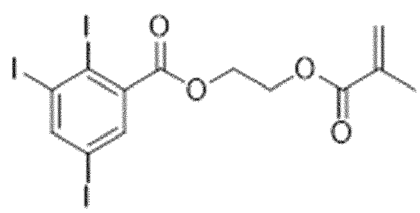

MAOETIB

La radio-opacité se réfère à l'incapacité relative de l'électromagnétisme, en particulier des rayons X, à traverser des matériaux denses, qui sont décrits comme « radio-opaques » apparaissant opaque/blanc dans une image radiographique. Compte tenu de la complexité du contenu dans une image radiographique ou fluoroscopique, les cliniciens sont sensibles à la qualité de l'image en ce qui concerne la luminosité ou la puissance du signal du matériau dans l'image. Les deux facteurs principaux qui contribuent à l'importance de la radio-opacité, sont la densité et le nombre atomique. Les dispositifs médicaux à base de polymère nécessitant une radio-opacité utilisent typiquement un mélange de polymères qui incorpore une petite quantité, en pourcentage en poids, d'un élément radio-opaque comme par exemple un atome lourd tel qu'un halogène, en particulier l'iode. La capacité d'un dispositif à être visualisé par fluoroscopie

dépend de la quantité ou de la densité de l'élément radio-opaque mélangé dans le matériau. Toutefois, l'addition d'une entité ou d'un monomère radio-opaque possédant des groupements halogénés semble diminuer considérablement le caractère hydrophile du matériau. En outre, les microsphères intégrant ce type d'entités ou monomère radio-opaque voient leur densité augmentée, ce qui impacte leurs propriétés de suspendabilité dans le milieu d'injection. En résumé, les microsphères chargées en iode pour être visibles en RX de l'art antérieur sont typiquement plus hydrophobes, denses et rigides que les microsphères non visibles en RX et ont tendance à former des agrégats de microsphères. En conséquence, (1) elles sont difficiles à maintenir en suspension pendant la durée de l'injection dans le cathéter, et (2) elles bloquent souvent le cathéter, même quand leur diamètre est inférieur au diamètre interne du cathéter (Duran 2016), par exemple du fait qu'elles s'agglomèrent plus facilement entre elles (3) elles ont tendance à coller les parois du cathéter.

Il est donc préférable de disposer d'entités ou monomères radio-opaque entrant dans la composition des microsphères d'embolisation qui permettent à ces dernières de rester hydrophiles et souples lorsqu'elles sont gonflées d'eau. Il est également souhaité que ces microsphères présentent des propriétés mécaniques, en particulier un taux de gonflement, une élasticité et une compressibilité, adéquates pour une injection via un cathéter ou un microcathéter. Il est également souhaité que ces microsphères puissent être maintenues en suspension dans le mélange d'injection (mélange composé de produit de contraste et de phase aqueuse) pendant la durée de l'injection dans le cathéter. En effet, pour être injectables et pour que le praticien puisse suivre l'injection sous contrôle RX, les microsphères sont généralement mises en suspension dans un mélange de produit de contraste iodé non ionique et de phase aqueuse. Pour cela, les radiologues utilisent généralement une solution de produit de contraste et éventuellement de sérum physiologique, de tampon bicarbonate ou de tampon phosphate, avantageusement une solution de 100 % de produit de contraste. Pour garantir leur injectabilité, les microsphères doivent être maintenues en suspension de manière homogène dans cette solution. Si les microsphères sédimentent ou, au contraire, flottent à la surface de la solution, la suspension résultante est non homogène, instable et ne peut donc pas être injectée au patient.

Les demandes WO 2021 /069527 et WO 2021 /069528 décrivent des monomères radio-opaques halogénés qui permettent de répondre à ces exigences de manière satisfaisante. Toutefois, le besoin reste pour de nouvelles entités ou monomères radio-opaques destinés à la préparation de microsphères d'embolisation qui permettent d'obtenir de meilleures performances, par exemple en termes de stabilité ou d'injectabilité des suspensions comprenant lesdites microsphères, tout en restant compatibles avec les produits de contraste iodés tels que décrits ci-dessus.

## RESUME DE L'INVENTION

Dans ce contexte, les inventeurs ont mis au point un nouveau monomère radio-opaque halogéné de formule (A) conduisant à une amélioration des performances des microsphères d'embolisation comprenant ce monomère dans leur composition. Par exemple, ce nouveau monomère radio-opaque de formule (A) permet notamment aux microsphères d'embolisation le comprenant d'éviter de s'agréger entre elles dans le cathéter ou le microcathéter avant l'injection. La présence de ce nouveau monomère radio-opaque halogéné de formule (A) dans les microsphères d'embolisation empêche également ces dernières de coller aux parois du cathéter ou du microcathéter avant l'injection. En outre, les propriétés de suspendabilité et d'injectabilité de ces microsphères se trouvent améliorées grâce à ce nouveau monomère radio-opaque.

Par l'expression « suspendabilité améliorée», on entend, au sens de la présente invention, la capacité des microsphères à former une suspension stable dans un temps compatible avec leur utilisation, et homogène, c'est-à-dire avec une répartition des microsphères identique en tout point du volume de la suspension.

Par l'expression « propriétés d'injectabilité améliorées », on entend, au sens de la présente invention, la capacité de la suspension à être injectée via un système d'injection, comme une seringue ou un cathéter, sans créer de bouchons et sans nécessiter de force importante de la part du praticien.

La présente invention concerne donc un composé de formule (A) suivante :

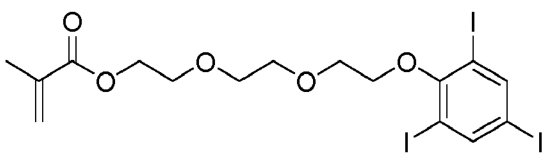
(A)

Ce composé est également désigné par le terme MAETIP dans la présente description.

Un autre objet de la présente invention concerne l'utilisation du composé de formule (A) tel que défini ci-dessus en tant que monomère halogéné radio-opaque.

Un autre objet de l'invention concerne des microsphères d'embolisation comprenant ledit monomère radio-opaque halogéné de formule (A).

La présente invention concerne donc également l'utilisation de ce composé de formule (I) dans des microsphères d'embolisation.

La présente invention porte en outre sur une composition pharmaceutique comprenant des microsphères d'embolisation telles que définies ci-dessus, en association avec un véhicule pharmaceutiquement acceptable, avantageusement pour une administration par injection.

La présente invention a également pour objet un kit comprenant une composition pharmaceutique telle que définie ci-dessus et au moins un moyen d'injection de ladite composition, pour une administration de ladite composition par voie parentérale.

La présente invention a également pour objet un kit comprenant d'une part une composition pharmaceutique telle que définie ci-dessus et d'autre part un agent de contraste pour l'imagerie par rayon X, par résonance magnétique ou par échographie, et éventuellement au moins un moyen d'injection pour une administration par voie parentérale, avantageusement ledit moyen d'injection est le dispositif Vectorio® tel que décrit dans les demandes WO2016/166346, WO2016/166339, WO2017/005914 et WO2017/081178.

DESCRIPTION DETAILLEE

Le principal objet de la présente invention est donc le composé de formule (A) suivante :

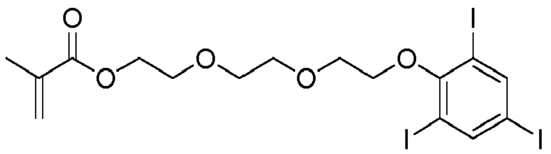

(A)

Dans le composé de formule (A), les atomes d'iode sont placés en position 2, 4 et 6 du cycle phényle. En raison de la taille des atomes d'iode et de leur répartition homogène sur le cycle phényle, ce composé présente une accessibilité spatiale réduite aux carbones aromatiques (en positions 3 et 5 du cycle phényle), par rapport au MAOETIB ou au composé (Vb) de la demande WO 2021 /069528 (où les atomes d'iode sont en position 2, 3 et 5 et les carbones aromatiques en position 4 et 6 du cycle phényle). L'accessibilité restreinte aux carbones aromatiques dans le composé de formule (A) semble avoir pour effet de diminuer le caractère lipophile de la molécule. En effet, les interactions inter- ou intramoléculaire de ces carbones sont réduites, voire nulle, de sorte à limiter le caractère collant de la molécule. En d'autres termes, la configuration spatiale du composé de formule (A) permet de limiter l'agrégation entre elles des microsphères d'embolisation intégrant ledit composé.

Selon la présente invention, ce composé de formule (A) est avantageusement utilisé en tant que monomère halogéné radio-opaque. Ainsi, la présente invention a également pour objet l'utilisation du composé de formule (A) tel que défini ci-dessus en tant que monomère halogéné radio-opaque.

En outre, la présente invention concerne des microsphères d'embolisation comprenant ledit monomère radio-opaque halogéné de formule (A). En particulier, lesdites microsphères d'embolisation comprennent une matrice polymérique réticulée comprenant le monomère radio-opaque halogéné de formule (A).

Dans un mode de réalisation particulier, ladite matrice polymérique réticulée est telle que définie dans la demande WO2021 /069528 à l'exception du monomère radio-opaque halogéné de formule générale (II) remplacé par le composé de formule (A) selon l'invention. En d'autres termes, ladite matrice polymérique réticulée est à base d'au moins :

a) 20 % à 90 % de monomère hydrophile choisi parmi la N-vinylpyrrolidone et un monomère de formule (I) suivante :

$$(CH_2{=}CR_1)\text{-}CO\text{-}D \qquad (I)$$

dans laquelle :

- D représente O-Z ou NH-Z, Z représentant $(C_1\text{-}C_6)$alkyle, $-(CR_2R_3)_m\text{-}CH_3$, $-(CH_2\text{-}CH_2\text{-}O)_m\text{-}H$, $-(CH_2\text{-}CH_2\text{-}O)_m\text{-}CH_3$, $-C(R_4OH)_m$ ou $-(CH_2)_m\text{-}NR_5R_6$ avec m représentant un nombre entier de 1 à 30 , de préférence m est égal à 4 ou 5
- $R_1$, $R_2$, $R_3$, $R_4$, $R_5$ et $R_6$ représentent, indépendamment les uns des autres, H ou un $(C_1\text{-}C_6)$alkyle ;

b) 5 % à 50 % de composé de formule (A) suivante :

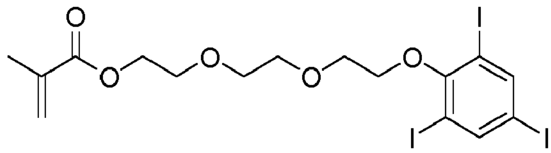 (A)

c) 1 % à 15 % de monomère réticulant hydrophile non biodégradable linéaire ou ramifié et présentant des groupes ($CH_2$=($CR_{16}$))- à chacune de ses extrémités, chaque $R_{16}$ représentant indépendamment H ou un ($C_1$-$C_6$)alkyle ; et
d) 0,1 % à 10 % d'agent de transfert choisi parmi les halogénures d'alkyle et les thiols cycloaliphatiques ou aliphatiques ayant notamment de 2 à 24 atomes de carbone, et ayant éventuellement un autre groupe fonctionnel choisi parmi les groupes amino, hydroxy et carboxy,
les pourcentages des monomères a) à c) étant cités en mole par rapport au nombre de moles total de monomères et les pourcentages du composé d) étant cités en mole par rapport au nombre de moles du monomère hydrophile a).

Le monomère hydrophile de formule (I), le monomère réticulant c) et l'agent de transfert d) sont avantageusement tels que définis dans la demande WO2021/069528, en particulier aux pages 13 et 19-22.

Par « monomère hydrophile », on entend, au sens de la présente invention, un monomère ayant une forte affinité pour l'eau, c'est-à-dire tendant à se dissoudre dans l'eau, à se mélanger avec l'eau, à être mouillé par l'eau, ou capable de gonfler dans l'eau après polymérisation.

Par « monomère réticulant », on entend, au sens de la présente invention, un monomère au moins bifonctionnel mais aussi multifonctionnel possédant une double liaison à chaque extrémité polymérisable. Le monomère réticulant, en combinaison avec les autres monomères dans le mélange, permet la formation d'un réseau réticulé. La structure et la quantité de monomère(s) réticulant(s) dans le mélange de monomères peuvent être choisies aisément par l'homme du métier pour fournir la densité de réticulation souhaitée. Le réticulant est également avantageux pour la stabilité des microsphères. Le réticulant empêche que les microsphères puissent se dissoudre dans n'importe quel solvant. Le réticulant permet également d'améliorer la compressibilité des microsphères, ce qui est favorable à l'embolisation.

Par « réticulant hydrophile non-biodégradable », on entend, au sens de la présente invention, un réticulant tel que défini ci-dessus, ayant une forte affinité pour l'eau et ne pouvant être dégradé dans les conditions physiologiques du corps d'un mammifère, en particulier du corps humain. En effet, la biodégradation d'une molécule est permise lorsque celle-ci contient suffisamment de sites fonctionnels pouvant être clivés dans les conditions physiologiques, en particulier par les enzymes endogènes du corps d'un mammifère, notamment du corps humain, et/ou à pH physiologique (généralement aux environs de 7,4). Les sites fonctionnels clivables dans les conditions physiologiques sont notamment les liaisons amides, les liaisons ester et les acétals. Une molécule comprenant un nombre insuffisant desdits sites fonctionnels sera donc considérée comme non biodégradable. Dans le contexte de la présente invention, le monomère réticulant contient moins de 20 sites fonctionnels clivables dans les conditions physiologiques, de préférence, moins de 15 sites, plus préférablement moins de 10 sites, encore plus préférablement moins de 5 sites.

Dans le cadre de la présente invention, on entend par « agent de transfert », un composé chimique possédant au moins une liaison chimique faible. Cet agent réagit avec le site radicalaire d'une chaîne polymère en croissance et interrompt la croissance de la chaîne. Dans le processus de transfert de chaîne, le radical est transféré temporairement à l'agent de transfert qui relance la croissance en transférant le radical à un autre polymère ou monomère.

Par l'expression « matrice à base de », il faut bien entendu comprendre une matrice comportant le mélange et/ou le produit de la réaction entre les constituants de bases utilisés pour la polymérisation en milieu hétérogène de cette matrice, de préférence uniquement le produit de la réaction entre les différents constituants de base utilisés pour cette matrice, certains d'entre eux pouvant être destinés à réagir ou susceptibles de réagir entre eux ou avec leur environnement chimique proche, au moins en partie, lors des différentes phases du procédé de fabrication de la matrice, en particulier au cours d'une étape de polymérisation. Ainsi, les constituants de base sont les réactifs destinés à réagir ensemble lors de la polymérisation de la matrice. Les constituants de bases sont donc introduits dans un mélange réactionnel comprenant éventuellement en outre un solvant ou un mélange de solvants et/ou d'autres additifs tels que au moins un sel et/ou au moins un amorceur de polymérisation et/ou au moins un stabilisant tel que le PVA. Dans le cadre de la présente invention le mélange réactionnel comprend au moins les monomères a), b), c) et l'agent de transfert d) cités dans la présente description en tant que constituants de base, éventuellement un amorceur de polymérisation comme par exemple le peroxyde de t-butyle, le peroxyde de benzoyle, l'acide azobiscyanovalerique (aussi nommé acide 4,4'-Azobis(4-cyanopentanoique)), l'AIBN (azobisisobutyronitrile), ou 1,1'- Azobis(cyclohexane carbonitrile) ou un ou plusieurs amorceurs thermiques tels que 2-Hydroxy-4'-(2-hydroxyethoxy)-2-methylpropiophenone (106797-53-9); 2-Hydroxy-2-methylpropiophenone (Darocur® 1173, 7473-98-5); 2,2-Dimethoxy-2-phenylacetophenone (24650-42-8); 2,2-dimethoxy-2-phenyl acetophenone (Irgacure®, 24650-42-8) ou 2-Methyl-4'-(methylthio)-2-morpholinopropiophenone (Irgacure®, 71868-10-5), et au moins un solvant, de préférence un mélange de solvant comprenant un solvant aqueux

et un solvant organique tel qu'un solvant aprotique apolaire, par exemple un système non miscible eau/toluène.

Ainsi, selon la présente invention, la matrice est au moins à base des monomères a), b), c) et de l'agent de transfert d) cités dans la présente description, ces composés étant donc des constituants de base.

Ainsi, dans la présente description, les expressions semblables à « le [constituant de base X] est en particulier ajouté dans le mélange réactionnel en une quantité de YY % à YYY % » et à « la matrice réticulée est en particulier à base du [constituant de base X] en une quantité de YY % à YYY % » sont interprétées de manière similaire. De même, les expressions semblables à « le mélange réactionnel comprend au moins [le constituant de base X] » et à « la matrice réticulée est à base d'au moins [le constituant de base X] » sont interprétées de manière similaire.

Par « phase organique » du mélange réactionnel, on entend, au sens de la présente invention, la phase comprenant le solvant organique et les composés solubles dans ledit solvant organique, notamment les monomères, l'agent de transfert et l'amorceur de polymérisation.

Par groupement « $(C_X-C_Y)$alkyle », on entend, au sens de la présente invention, une chaîne hydrocarbonée monovalente saturée, linéaire ou ramifiée, comportant X à Y atomes de carbone, X et Y étant des nombres entiers compris entre 1 et 36, de préférence 1 et 18, en particulier 1 et 6. A titre d'exemple, on peut citer les groupes méthyle, éthyle, propyle, isopropyle, butyle, isobutyle, sec-butyle, tert-butyle, pentyle ou encore hexyle.

Dans le cadre de la présente invention, le composé de formule (A) est en particulier ajouté dans le mélange réactionnel en une quantité de 5 % à 50%, en particulier en une quantité supérieure à 7 % et inférieure ou égale à 50 %, en particulier en une quantité supérieure à 10 % et inférieure ou égale à 50 %, plus particulièrement en une quantité supérieure à 15 % et inférieure ou égale à 50 %, de préférence en une quantité supérieure à 15 % et inférieure ou égale à 35 %, et en particulier de 20 % à 30 % par Mole, par rapport au nombre de moles total de monomères.

Les microsphères d'embolisation comprenant la matrice polymérique réticulée telle que définie ci-dessus correspondent avantageusement à des particules sphériques ayant un diamètre après gonflement allant de 20 à 1200 μm, par exemple de 20 à 100 μm, de 40 à 150 μm, de 100 à 300 μm, de 300 à 500 μm, de 500 à 700 μm, de 700 à 900 μm ou de 900 à 1200 μm, telle que déterminé par microscopie optique. Les microsphères ont avantageusement un diamètre suffisamment petit pour être injectées par des aiguilles, un cathéter ou un microcathéter de diamètre interne variant de quelques centaines de micromètres à plus d'un millimètre.

L'expression « après gonflement » signifie que la taille des microsphères est considérée après les étapes de polymérisation et de stérilisation qui interviennent lors de leur préparation. L'étape de stérilisation implique par exemple un passage des microsphères après l'étape de polymérisation dans un autoclave à haute température, typiquement à une température supérieure à 100°C, de préférence à une température comprise entre 110 °C et 150°C, de préférence 121°C. Lors de cette étape de stérilisation, les microsphères continuent de gonfler de manière contrôlée, c'est-à-dire avec un taux de gonflement maitrisé. Le taux de gonflement est défini comme :

$$taux\ de\ gonflement\ massique\ (Q) = \frac{m_w(g) - m_d(g)}{m_{d\ (g)}}$$

où $m_w$ est le poids en gramme de 1 mL de microsphères sédimentées et $m_d$ est le poids en gramme de 1ml de microsphères sédimentées qui ont été ensuite lyophilisées.

Dans un mode de réalisation particulier selon l'invention, la matrice polymérique réticulée des microsphères est uniquement à base des constituants de base a), b), c) et d) tels que définis ci-dessus, dans les proportions susmentionnées de monomères et d'agent de transfert, aucun autre constituant de base n'étant ajouté au milieu réactionnel. Il est ainsi manifeste que la somme des proportions susmentionnées de monomères a), b) et c) doit être égale à 100 %.

De manière préférée, le monomère hydrophile de formule (I) est choisi dans le groupe constitué de N-vinylpyrrolidone, alcool vinylique, 2-hydroxyéthylméthacrylate, acrylate de sec-butyle, acrylate de n-butyle, acrylate de t-butyle, méthacrylate de t-butyle, méthylméthacrylate, N-diméthylaminoéthyl(méthyl)acrylate, N,N-diméthylaminopropyl-(méth)acrylate, *t*-butylaminoéthyl(méthyl)acrylate, N,N-diéthylaminoacrylate, (méth)acrylate de poly(éthylène oxyde), (méth)acrylate de méthoxy poly(éthylène oxyde), (méth)acrylate de butoxy poly(éthylène oxyde), (méth)acrylate de poly(éthylène glycol), (méth)acrylate de méthoxy poly(éthylène glycol), (méth)acrylate de butoxy poly(éthylène glycol), méthyl éther méthacrylate de poly(éthylène glycol) et leurs mélanges.

Plus avantageusement, le monomère hydrophile a) est le méthyl éther méthacrylate de poly(éthylène glycol) (m-PEGMA).

Dans le cadre de la présente invention, le monomère hydrophile a) est en particulier ajouté dans le mélange réactionnel en une quantité de 20 % à 90%, de préférence 30 % à 80%, de manière préférée de 40 % à 70%, en particulier de 45 % à 65% par Mole, par rapport au nombre de moles total de monomères. Ainsi, dans le cadre de la présente invention, la matrice réticulée en en particulier à base du monomère hydrophile a) en une quantité de 20 % à 90%, de préférence 30 % à 80%, de manière préférée de 40 % à 70%, en particulier de 45 % à 65% par Mole, par rapport au nombre de moles total de monomères.

Avantageusement, le monomère réticulant hydrophile non biodégradable linéaire ou ramifié présente des groupes ($CH_2$=($CR_{16}$))CO- ou ($CH_2$=($CR_{16}$))CO-O- à ses au moins deux extrémités, chaque $R_{16}$ représentant indépendamment H ou un ($C_1$-$C_6$)alkyle.

De manière particulière, le réticulant est de formule générale (IIIa) ou (IIIb) suivante :

($CH_2$=($CR_{16}$))CO-NH-A-HN-OC(($CR_{16}$)=$CH_2$) (IIIa),

($CH_2$=($CR_{16}$))CO-O-A-O-OC(($CR_{16}$)=$CH_2$) (IIIb),

dans lesquelles

chaque $R_{16}$ représente indépendamment H ou un ($C_1$-$C_6$)alkyle, avantageusement les radicaux $R_{16}$ sont identiques et représentent H ou ($C_1$-$C_6$)alkyle ; et

A représente, seul ou avec au moins un des atomes auquel il est lié, un ($C_1$-$C_6$)alkylène, un polyéthylène glycol (PEG), un polysiloxane, un poly(diméthylsiloxane) (PDMS), un polyglycérolique ester (PGE) ou un bisphénol A.

Avantageusement, le réticulant est de formule générale (IIa) ou (IIb) suivante :

($CH_2$=($CR_{16}$))CO-NH-A-HN-OC(($CR_{16}$)=$CH_2$) (IIIa),

($CH_2$=($CR_{16}$))CO-O-A-O-OC(($CR_{16}$)=$CH_2$) (IIIb),

dans lesquelles,

chaque $R_{16}$ représente indépendamment H ou un ($C_1$-$C_6$)alkyle, avantageusement les radicaux $R_{16}$ sont identiques et représentent H ou ($C_1$-$C_6$)alkyle ; et

A représente préférablement, seul ou avec au moins un des atomes auquel il est lié, un ($C_1$-$C_6$)alkylène ou un polyéthylène glycol (PEG), de préférence un polyéthylène glycol (PEG).

Dans le cadre des définitions de A ci-dessus, le polyéthylène glycol a une longueur variant de 200 à 10 000 g/mol, de préférence de 200 à 2 000 g/mol, plus préférablement de 500 à 1 000 g/mol.

Comme exemple de monomère réticulant pouvant être utilisé dans le cadre de la présente invention, on peut citer (sans être limitatif) : le 1,4-butanediol diacrylate, le pentaérythritol tétraacrylate, le méthylènebisacrylamide, le glycérol 1,3-diglycérolate diacrylate et le poly(éthylène glycol)diméthacrylate (PEGDMA).

Avantageusement, le monomère réticulant est le poly(éthylène glycol)diméthacrylate (PEGDMA), le motif polyéthylène glycol ayant une longueur variant de 200 à 10 000 g/mol, de préférence de 200 à 2 000 g/mol, plus préférablement de 500 à 1 000 g/mol.

Dans le cadre de la présente invention, le monomère réticulant est en particulier ajouté dans le mélange réactionnel en une quantité de 1 % à 15%, de préférence de 2 % à 10%, en particulier 2 % à 7 %, plus particulièrement 2 % à 5 % par mole, par rapport au nombre de moles total de monomères.

Avantageusement, ledit agent de transfert de chaine est choisi dans le groupe constitué par les thiols monofonctionnels ou polyfonctionnels, et les halogénures d'alkyle.

Parmi les halogénures d'alkyle pouvant servir d'agent de transfert, on retrouve en particulier le bromotrichlorométhane, le tétrachlorométhane et le tétrabromométhane. De manière particulièrement avantageuse, ledit agent de transfert de chaine est un thiol cycloaliphatique ou aliphatique ayant typiquement de 2 à environ 24 atomes de carbone, de préférence 2 à 12 atomes de carbone, plus préférentiellement 6 atomes de carbone, et ayant éventuellement un groupe fonctionnel supplémentaire choisi parmi les groupes amino, hydroxy et carboxy.

Avantageusement, l'agent de transfert est choisi parmi l'acide thioglycolique, le 2-mercaptoéthanol, le dodécanethiol, l'hexanethiol et leurs mélanges.

Dans le cadre de la présente invention, l'agent de transfert est en particulier ajouté dans le mélange réactionnel en une quantité de 0,1 % à 10%, de préférence de 0,5 % à 8%, plus avantageusement de 1,5 % à 6 % et en particulier de 1,5 % à 4,5% par Mole, et en particulier de 3% par mole, par rapport au nombre de moles de monomère hydrophile a).

Dans un autre mode de réalisation particulier de l'invention, la matrice réticulée polymérique des microsphères de l'invention est en outre à base :

- d'un monomère ionisé ou ionisable et/ou

- d'un monomère coloré pour les rendre visibles à l'œil nu, afin par exemple de vérifier avant injection que la suspension de microsphères est bien homogène dans la seringue et de contrôler la vitesse d'injection, et/ou
- d'au moins un agent visible en imagerie par résonance magnétique (IRM)

Lesdits monomère ionisé ou ionisable, coloré et agent visible en IRM sont notamment tels que définis dans la demande WO2021/069528, en particulier aux pages 22-25.

En particulier, la matrice réticulée polymérique des microsphères d'embolisation de l'invention peut en outre être à base d'au moins un monomère ionisé ou ionisable de la formule (IV) suivante :

$$(CH_2{=}CR_{17})\text{-M-E} \qquad (IV)$$

dans laquelle :

- $R_{17}$ représente H ou un ($C_1$-$C_6$) alkyle ;
- M représente une liaison simple ou un radical divalent ayant de 1 à 20 atomes de carbone ;
- E représente un groupe chargé ou ionisable ayant 100 atomes au plus, E étant avantageusement choisi dans le groupe constitué de -COOH, -COO-, $-SO_3H$, $-SO_3^-$, $-PO_3H_2$, $-PO_3H^-$, $-PO_3^{2-}$, $-NR_{18}R_{19}$, $-NR_{20}R_{21}R_{22}^+$,
- $R_{18}$, $R_{19}$, $R_{20}$, $R_{21}$ et $R_{22}$ représentent indépendamment les uns des autres H ou un ($C_1$-$C_6$)alkyle.

Par groupement ionisé ou ionisable, on entend, au sens de la présente invention, un groupement chargé ou pouvant se trouver sous forme chargée (sous forme d'ion), c'est-à-dire portant au moins une charge positive ou négative, selon le pH du milieu. Par exemple, le groupement COOH pourra être ionisé sous forme $COO^-$ et le groupement $NH_2$ peut se trouver sous forme ionisée $NH_3^+$.

L'introduction d'un monomère ionisé ou ionisable dans le mélange réactionnel permet d'augmenter l'hydrophilie des microsphères résultantes, augmentant ainsi le taux de gonflement desdites microsphères, facilitant davantage leur injection via les cathéters et microcathéters. De plus, la présence d'un monomère ionisé ou ionisable permet le chargement de substances actives au sein de la microsphère.

Selon une variante préférée, le monomère ionisé ou ionisable est de formule (IV-A) suivante :

$$(CH_2{=}CR_{17})\text{-C(O)-O-M'-E} \qquad (IV)$$

dans laquelle :

- $R_{17}$ et E sont tels que définis ci-dessus, et
- M' une chaîne hydrocarbonée comprenant de 1 à 20 atomes.

De préférence, le monomère ionisé ou ionisable est un monomère cationique, avantageusement choisi dans le groupe consistant en l'acide méthacrylique, le (méthacryloyloxy)éthylphosphorylcholine, le (méth)acrylate de 2-(diméthylamino)éthyle, le (méth)acrylate de 2-(diéthylamino)éthyle), l'acide11-méthacryloyloxyundecylphosphonique et le chlorure de 2-((méth)acryloyloxy)éthyl)-triméthylammonium, avantageusement, le monomère cationique est le (méth)acrylate de diéthylamino)éthyle. Avantageusement, la matrice réticulée selon l'invention est à base d'un monomère cationique mentionné ci-dessus en des quantités comprises entre 1 % et 40% en moles par rapport au nombre de moles total de monomères. De préférence, la matrice réticulée selon l'invention est à base de monomère ionisé ou ionisable en des quantités comprises entre 5 % et 15 %, de préférence 10 % en moles par rapport au nombre de moles total de monomères, lorsque les microsphères résultantes ne sont pas destinées à être chargées avec une substance active. Selon un autre mode de réalisation, lorsque les microsphères sont destinées à être chargées avec une substance active, la matrice réticulée selon l'invention est obtenue en ajoutant au mélange réactionnel entre 20 % et 40 %, de préférence en ajoutant au mélange réactionnel 20 % à 30% en moles de monomère ionisé ou ionisable par rapport au nombre de moles total de monomères.

Dans un autre mode de réalisation avantageux, le monomère ionisé ou ionisable, est un monomère anionique avantageusement choisi dans le groupe constitué par l'acide acrylique, l'acide méthacrylique, l'acrylate de 2-carboxyéthyle, les 2-oligomères d'acrylate de carboxyéthyle, le (méth)acrylate de 3-sulfopropyle, le sel de potassium et l'hydroxyde de 2-((méthacryloyloxy)éthyl)diméthyl-(3-sulfopropyl)ammonium. Avantageusement, la matrice réticulée selon l'invention est à base d'un monomère anionique mentionné ci-dessus en des quantités comprises entre 1 % et 40% en moles sur la base de la quantité totale de monomères. De préférence, la matrice réticulée selon l'invention est à base de monomère ionisé ou ionisable en des quantités comprises entre 5 % et 15 %, de préférence 10 % en moles sur la base de la quantité totale de monomères, lorsque les microsphères résultantes ne sont pas destinées à être chargées avec une substance active. Selon un autre mode de réalisation, lorsque les microsphères sont destinées à être chargées

avec une substance active, la matrice réticulée selon l'invention est à base de monomère ionisé ou ionisable en des quantités comprises entre 20 % et 40 %, de préférence 20 % à 30% de monomère ionisé ou ionisable sur la base de la quantité totale de monomères.

De manière particulièrement avantageuse, le monomère ionisé ou ionisable, est l'acide méthacrylique (MA ou AM). Avantageusement, la matrice réticulée selon l'invention est à base d'acide méthacrylique (MA) en des quantités comprises entre 10 % et 30% en moles sur la base de la quantité totale de monomères.

Ladite matrice polymérique réticulée peut en outre être à base d'au moins un monomère coloré de formule générale (VI) suivante :

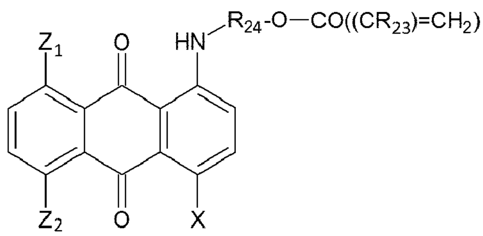

(VI),

dans laquelle,

- $Z_1$ et $Z_2$ représentent, indépendamment l'un de l'autre, H ou $OR_{25}$, $R_{25}$ représentant H ou un ($C_1$-$C_6$)alkyle, avantageusement $Z_1$ et $Z_2$ représentent H ;
- X représente H ou Cl, avantageusement H ;
- $R_{23}$ représente H ou un ($C_1$-$C_6$)alkyle, avantageusement un ($C_1$-$C_6$)alkyle, en particulier un méthyle ; et
- $R_{24}$ représente un groupe choisi parmi ($C_1$-$C_6$)alkylène linéaire ou ramifié, ($C_5$-$C_{36}$)arylène, ($C_5$-$C_{36}$)arylène-O-$R_{26}$, ($C_5$-$C_{36}$)hétéroarylène et ($C_5$-$C_{36}$)hétéroarylène-O-$R_{27}$, $R_{26}$ et $R_{27}$ représentant un ($C_1$-$C_6$)alkyle ou un ($C_1$-$C_6$)alkylène, avantageusement $R_{24}$ représente un groupe $-C_6H_4-O-(CH_2)_2-O$ ou $-C(CH_3)_2-CH_2-O$.

Par groupement « ($C_X$-$C_Y$)alkylène », on entend, au sens de la présente invention, une chaîne hydrocarbonée divalente, linéaire ou ramifiée, comprenant X à Y atomes de carbone, X et Y étant des nombres entiers compris entre 1 et 36, de préférence 1 et 18, en particulier 1 et 6. A titre d'exemple, on peut citer les groupements methylène, éthylène, propylène, butylène, pentylène ou encore hexylène.

Par « ($C_X$-$C_Y$)hétéroarylène », on entend, au sens de la présente invention, un groupement divalent aromatique, comportant de X à Y atomes cycliques dont un ou plusieurs hétéroatomes, avantageusement 1 à 4 et encore plus avantageusement 1 ou 2, tels que par exemple des atomes de soufre, azote ou oxygène, les autres atomes cycliques étant des atomes de carbone. X et Y sont des nombres entiers compris entre 5 et 36, de préférence 5 et 18, en particulier 5 et 10.

Par « radical divalent », on entend, au sens de la présente invention, un radical ayant une valence de 2, c'est-à-dire ayant deux liaisons chimiques covalentes, covalentes polaires ou ioniques. Ledit radical peut comprendre par exemple des atomes de carbone et/ou d'oxygène.

Avantageusement, le monomère coloré est de formule (VIa) ou (VIb) suivante :

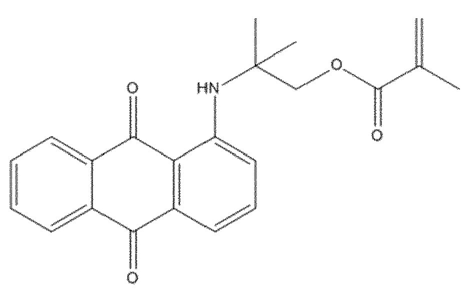

(VIa)

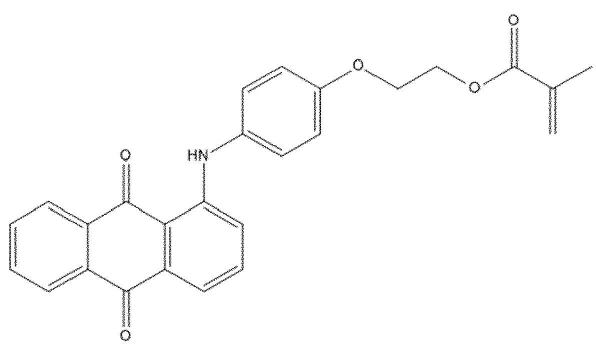

(VIb).

Plus avantageusement, le monomère coloré est de formule (VIb) ci-dessus.

Dans le cadre de la présente invention, le monomère coloré est en particulier ajouté dans le mélange réactionnel en une quantité de 0 % à 1%, de préférence de 0 % à 0,5 %, plus particulièrement de 0,02 % à 0,2 %, et encore plus

particulièrement de 0,04 % à 0,1% par Mole, par rapport au nombre de moles total de monomères.

Ladite matrice polymérique réticulée peut en outre être à base d'éléments visibles en imagerie par résonance magnétique (IRM) telles que des nanoparticules d'oxyde de fer, des chélates de gadolinium ou des chélates de magnésium, avantageusement des nanoparticules d'oxyde de fer.

Dans le cadre de la présente invention, les éléments visibles en IRM sont avantageusement ajoutés dans le mélange réactionnel en une quantité de 0 % à 0,5%, de préférence de 0,025 % à 0,4 %, plus préférentiellement de 0,025 % à 0,25%, notamment 0,05 %, en masse d'élément visible en IRM par volume de phase organique, de sorte à obtenir des microsphères visibles et quantifiables par IRM.

La matrice réticulée polymérique des microsphères d'embolisation de l'invention peut être facilement synthétisée par de nombreux procédés bien connus de l'homme de l'art. A titre d'exemple, elle peut être obtenue par polymérisation en suspension comme décrit dans la demande WO2021/069528, en particulier aux pages 27-29.

Dans un mode de réalisation particulier, les microsphères d'embolisation selon la présente invention sont chargées de substances actives, permettant ainsi de combiner l'occlusion vasculaire et la délivrance d'un principe actif. Ladite substance active peut être choisie parmi un médicament, un agent de diagnostic et des macromolécules telles que définies dans la demande WO2021/069528, notamment aux pages 29-31.

De préférence, les microsphères selon l'invention peuvent être chargées d'une substance active choisie parmi les agents anticancéreux, les agents anti-inflammatoires, les anesthésiques locaux, les analgésiques, les antibiotiques, les stéroïdes, les antiseptiques et leurs mélanges.

L'agent anticancéreux est préférentiellement choisi parmi les anthracyclines telle que la doxorubicine, l'epirubicine ou l'idarubicine, les complexes de platine, les composés apparentés aux anthracyclines tels que la mitoxantrone et la nemorubicine, les antibiotiques tels que la mitomycine C (Ametycine®), la bléomycine et l'actinomycine D, les autres composés anti-néoplasiques tels que l'irinotecan, le 5-Fluoro-Uracile (Adrucil®), le sorafénib (Nevaxar®), le sunitinib (Sutent®), le regorafénib, le brivanib, l'orantinib, le linsitinib, l'erlotinib, le cabozantinib, le foretinib, le tivantinib, la fotémustine, la tauromustine (TCNU), la carmustine, la cytosine C, le cyclophosphonamide, la cytosine arabinoside (ou cytarabine), le paclitaxel, le docétaxel, le methotrexate, l'everolimus (Afinitor®), le PEGarginine deiminase, la combinaison tegafur/gimeracil/oteracil (Teysuno®), le muparfostat, le pérétinoine, la gemcitabine, le bévacizumab (Avastin®), le ramucirumab, la floxuridine, les immunostimulants tels que le GM-CSF (Granulocyte-macrophage colony-stimulating factor) et ses formes recombinantes : molgramostim ou sargramostim (Leukine®), l'OK-432 (Picibanil®), l'interleukine-2, l'interleukine-4 et le Tumor necrosing Factor-alpha (TNFalpha), les anticorps, les radio-éléments, les complexes de ces radioéléments avec des chélates, les séquences d'acide nucléique et un mélange d'un ou plusieurs de ces composés (préférentiellement un mélange d'une ou plusieurs anthracyclines).

Préférentiellement, l'agent anticancéreux est choisi parmi les anthracyclines, les immunostimulants, les complexes de platine, les anti-néoplasiques et leurs mélanges.

Encore plus préférentiellement, l'agent anticancéreux est choisi parmi les anthracyclines, les anticorps, les anti-néoplasiques et leurs mélanges.

Les anticorps sont par exemple choisis parmi les anti-PD-1, les anti-PD-L1, les anti-CTLA-4, les anti-CEA (CarcinoEmbryonic Antigen) ou un mélange de ceux-ci.

Les anti-PD-1 sont par exemple le nivolumab ou le pembrolizumab.

Les anti-PD-L1 sont par exemple l'avelumab, le durvalumab ou l'atezolizumab.

Les anti-CTLA-4 sont par exemple l'ipilimumab ou le tremelimumab.

Encore plus avantageusement, l'anticancéreux est choisi dans le groupe constitué de paclitaxel, doxorubicine, epirubicine, idarubicine, irinotécan, GM-CSF (Granulocyte-macrophage colony-stimulating factor), tumor necrosing Factor-alpha (TNFalpha), les anticorps, et leurs mélanges.

Préférentiellement, l'anesthésique local est choisi parmi la lidocaïne, la bupivacaïne et leurs mélanges.

L'anti-inflammatoire peut être choisi parmi l'ibuprofène, l'acide niflumique, la dexaméthasone, le naproxène et leurs mélanges.

Dans le cadre de la présente invention, les microsphères peuvent être chargées, notamment par adsorption extemporanée, de macromolécules choisies dans le groupe constitué d'enzymes, d'anticorps, de cytokines, de facteurs de croissance, de facteurs de coagulation, d'hormones, de plasmides, d'oligonucléotides antisens, de siARN, de ribozymes, de DNA enzyme (aussi appelée DNAzyme), d'aptamères, de protéines anti-inflammatoires, des protéines morphogènes osseuses (BMP), des facteurs pro-angiogéniques, des facteurs de croissance endothéliale vasculaire (VEGF) et des TGF-bêta, et des inhibiteurs de l'angiogenèse ou des anti-tyrosine kinases et leurs mélanges.

Les protéines anti-inflammatoires sont par exemple l'infliximab ou le rilonacept et leur mélange.

Les facteurs pro-angiogéniques sont par exemple des facteurs de croissance des fibroblastes (FGF) et leur mélange.

Les inhibiteurs de l'angiogenèse sont par exemple le bevacizumab, le ramucirumab, le nesvacumab, l'olaratumab, le vanucizumab, le rilotumumab, l'emibetuzumab, l'aflibercept, le ficlatuzumab, le pegaptanib et leurs mélanges.

Les anti-tyrosine kinases sont par exemple le lenvatinib, le sorafenib, le sunitinib, le pazopanib, le vandetanib,

l'axitinib, le regorafenib, le cabozantinib, le fruquintinib, le nintedanib, l'anlotinib, le motesanib, le cediranib, le sulfatinib, le dovetinib, le linifanib et leurs mélanges.

De manière avantageuse, les microsphères peuvent être chargées de macromolécules choisies parmi les anti-tyrosine kinases, les TGF-bêta, les inhibiteurs de l'angiogenèse et leurs mélanges.

La substance active est typiquement adsorbée sur la matrice réticulée par interactions noncovalentes, éventuellement en présence d'excipient(s) pharmaceutiquement acceptable(s) bien connu(s) de l'homme du métier. Cette façon particulière de piéger les substances actives s'appelle l'encapsulation physique. Aucune exigence particulière n'est imposée sur la substance active à charger.

Le chargement peut se faire par de nombreux procédés bien connus de l'homme de l'art tels que l'adsorption passive (gonflement de la matrice réticulée dans une solution de médicament) ou par interaction ionique. Ces méthodes sont par exemple décrites dans la demande internationale WO 2012/120138, notamment de la page 22 ligne 20 à la page 26 ligne 7. L'efficacité du chargement dépend principalement de la compatibilité entre les deux structures et/ou des interactions favorables.

Un autre objet de l'invention porte sur une composition pharmaceutique comprenant des microsphères d'embolisation selon l'invention, en association avec un véhicule pharmaceutiquement acceptable, avantageusement pour une administration par injection.

Un exemple de véhicule pharmaceutiquement acceptable comprend, mais sans s'y limiter, de l'eau pour injection, de la solution saline également appelée sérum physiologique, de l'amidon, de l'hydrogel, de la polyvinylpyrrolidone, du polysaccharide, de l'ester d'acide hyaluronique, du plasma, un agent de contraste pour l'imagerie par rayon X, par résonance magnétique ou par échographie, un agent tampon, un conservateur, un agent gélifiant, du glucose et/ou un agent tensioactif. Avantageusement, le véhicule pharmaceutiquement acceptable est du sérum physiologique, de l'eau pour injection, un agent de contraste pour l'imagerie par rayon X, par résonance magnétique ou par échographie, ou leurs mélanges. Plus avantageusement, le véhicule pharmaceutiquement acceptable est du sérum physiologique, un agent de contraste pour l'imagerie par rayon X, par résonance magnétique ou par échographie, ou un mélange de sérum physiologique et d'un agent de contraste pour l'imagerie par rayon X, par résonance magnétique ou par échographie.

Selon la présente invention, l'agent de contraste est préférablement un agent de contraste pour l'imagerie par rayon X. Il s'agit avantageusement d'un agent de contraste hydrosoluble iodé non ionique, tel que par exemple l'iobitridol (Xenetix®), l'iopamidol (Iopamiron®, Isovue®), l'iomeprol (Iomeron®), l'ioversol (Optiray®, Optiject®), l'iohexol (Omnipaque®), l'iopentol (Imagopaque®), l'ioxitol (Oxilan®), l'iopromide (Ultravist®), le metrizamide (Amipaque®), l'iosarcol (Melitrast®), l'iotrolan (Isovist®), l'iodixanol (Visipaque®), l'iosiménol et l'iosimide (Univist®) et un mélange de ceux-ci.

Selon un autre mode de réalisation, l'agent de contraste est un agent de contraste pour l'imagerie par résonnance magnétique (IRM). Il s'agit avantageusement de chélates de gadolinium (Dotarem®, Gadopiclenol).

Selon un autre mode de réalisation, l'agent de contraste est un agent de contraste pour l'imagerie par échographie. Il s'agit avantageusement d'hexafluorure de soufre (Sonovue®).

Dans un mode de réalisation particulier de la présente invention, la composition pharmaceutique comprend des microsphères d'embolisation selon l'invention, en association avec du sérum physiologique, ladite composition étant destinée à être mélangée avec au moins un agent de contraste pour l'imagerie par rayon X, par résonance magnétique ou par échographie tel que défini ci-dessus, en particulier pour l'imagerie par rayon X, avant administration par injection, un tel mélange entrainant la mise en suspension des microsphères selon l'invention.

Dans un mode de réalisation particulier selon l'invention, la composition pharmaceutique selon l'invention comprend des microsphères d'embolisation selon l'invention, en association avec un mélange de sérum physiologique et d'un agent de contraste tel que défini ci-dessus, le sérum physiologique et l'agent de contraste étant présent dans des proportions 70/30 à 20/80, avantageusement de 50/50 à 20/80, de préférence 50/50.

La composition pharmaceutique doit avoir une viscosité acceptable pour l'injection.

Les microsphères d'embolisation selon l'invention peuvent, comme cela a été indiqué ci-dessus, être utilisées à diverses fins biomédicales, ce qui signifie qu'elles doivent être compatibles au corps humain ou au corps d'un mammifère. Plus particulièrement, les matériaux biomédicaux appropriés ne possèdent pas de propriétés hémolytiques.

La présente invention a également pour objet un kit comprenant une composition pharmaceutique telle que définie ci-dessus et au moins un moyen d'injection de ladite composition, pour une administration de ladite composition par voie parentérale. Selon la présente invention, on entend par « moyen d'injection » tout moyen permettant une administration par voie parentérale. Avantageusement, ledit moyen d'injection est une ou plusieurs seringues et/ou une ou plusieurs seringue(s) pouvant être préremplies et/ou un ou plusieurs cathéter(s) ou microcatheter(s) pour une administration de ladite composition par injection.

Avantageusement, la composition pharmaceutique présente dans ledit kit comprend les microsphères selon la présente invention en association avec du sérum physiologique, un agent de contraste, ou leur mélange. Plus avantageusement, ladite composition pharmaceutique comprend les microsphères selon la présente invention en association avec un mélange de sérum physiologique et d'un agent de contraste dans des proportions comprises entre 80/20 et

0/100, avantageusement comprises entre 70/30 et 40/60, préférentiellement 50/50.

Avantageusement, le moyen d'injection présent dans le kit selon l'invention est adapté pour l'administration par voie parentérale de la composition pharmaceutique selon l'invention. Ainsi, la taille de la(des) seringue(s) ou du(des) (micro)cathéter(s) sera adaptée en fonction de la taille des microsphères selon l'invention et du volume à injecter pour l'embolisation. L'homme du métier saura choisir le moyen d'injection adapté. Selon un mode de réalisation préféré, ledit moyen d'injection est le dispositif Vectorio® tel que décrit dans les demandes WO2016/166346, WO2016/166339, WO2017/005914 et WO2017/081178.

La présente invention a également pour objet un kit comprenant d'une part une composition pharmaceutique telle que définie ci-dessus et d'autre part au moins un agent de contraste pour l'imagerie par rayon X, par résonance magnétique ou par échographie, et éventuellement au moins un moyen d'injection pour une administration par voie parentérale. Le moyen d'injection est tel que défini ci-dessus.

Dans ledit kit, la composition pharmaceutique et l'agent de contraste sont conditionnés séparément et sont destinés à être mélangés juste avant l'administration par injection.

Dans ledit kit, l'au moins un agent de contraste est tel que défini ci-dessus dans la description. En particulier, l'au moins un agent de contraste est un agent de contraste pour l'imagerie par rayon X tel que défini ci-dessus dans la description.

Dans ledit kit, la composition pharmaceutique comprend avantageusement les microsphères selon la présente invention en association avec un véhicule pharmaceutiquement acceptable pour une administration par injection. Ledit véhicule pharmaceutiquement acceptable peut être par exemple, mais sans s'y limiter, de l'eau pour injection, du sérum physiologique, de l'amidon, de l'hydrogel, de la polyvinylpyrrolidone, du polysaccharide, de l'ester d'acide hyaluronique, du glucose et/ou du plasma. De préférence, Dans ledit kit, la composition pharmaceutique comprend avantageusement les microsphères selon la présente invention en association avec du sérum physiologique ou de l'eau pour injection.

Dans ledit kit, la composition pharmaceutique est avantageusement conditionnée directement dans un moyen d'injection, notamment dans une seringue, adapté pour l'injection de microsphères d'embolisation par voie parentérale.

Dans ledit kit, l'agent de contraste est avantageusement conditionné dans un flacon ou directement dans un moyen d'injection, notamment une seringue, en particulier adapté pour l'injection de microsphères d'embolisation par voie parentérale.

Dans ledit kit, les proportions véhicule pharmaceutiquement acceptable / agent de contraste sont comprises entre 50/50 et 0/100, avantageusement comprises entre 40/60 et 0/100, de préférence 30/70 à 0/100.

## EXEMPLES

### Exemple 1 : Synthèse du MAETIP (composé de formule (A) selon l'invention)

*Matériel et méthode*

Produits chimiques :

- Sulfate de magnésium ($MgSO_4$, anhydre, 98%, Sigma-Aldrich, ref: 230391, CAS : 10034-99-8)
- 2,4,6 - triiodophénol (95-98%, BLDpharm, ref : A17145, CAS : 609-23-4)
- 2-[2-(2-Chloroéthoxy) éthoxy] éthanol (95-98%, TCI, ref: QF-8470, CAS: 5197-62-6)
- Iodure de sodium (NaI, >99%, OakwoodChemical, ref : QE-0904, CAS : 7681-82-5)
- Hydroxide de sodium (NaOH, anhydre, >98%, Sigma-Aldrich, ref: S8045, CAS: 1310-73-2)
- Méthacrylate d'anhydride (AM, > 94%, Sigma-Aldrich, ref: 276685, CAS: 760-93-0)
- Triéthylamine (TEA, > 99,5%, Sigma-Aldrich, ref: 471283, CAS: 121-44-8)

Solvants:

- Ethanol absolu (EtOH, 99,96%, VWR, ref : 20821.310, CAS :64-17-5)
- Acetate d'ethyle (Anhydre, 99,8%, Sigma-Aldrich, ref :270989, CAS: 141-78-6)
- Heptane (>99%, Sigma-Aldrich, ref: 34873, CAS: 142-82-5)
- Dichlorométhane (DCM, anhydre, >99.8%, Sigma-Aldrich, ref: 270997, CAS: 75-09-2)
- Eau distillée (grade 2)

Equipements:

- Plaque magnétique (Heidoplh, MR Hei-Satndard)
- Evaporateur Rotatif (Buchi Rotavapor R-215)
- Balance de précision (d=0.1 mg, Sartorius)
- Colonne de silice : ChromatoFlash (Buchi)

1. O-alkylation

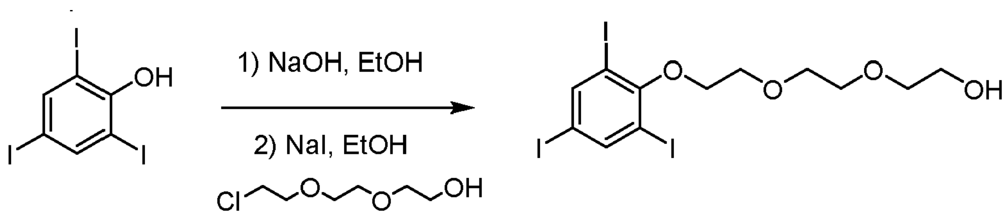


intermédiaire 1

Dans un ballon de 10 mL, le tri-iodophénol (200 mg ; 0.42 mmol) est solubilisé dans 2.2 mL d'éthanol. Le NaOH (15 mg ; 0.375 mmol) est ajouté, puis le mélange est agité 30 minutes à température ambiante. A la suite de l'agitation, on procède à une évaporation sous vide jusqu'à l'obtention d'un solide légèrement jaune.

Dans un tricol, équipé d'un réfrigérant, placé sous azote, on ajoute le NaI (57 mg ; 0.375 mmol) et le 2-[(2-Chloroéthoxy) éthoxy] éthanol (55 µL ; 0.375 mmol). Le mélange est solubilisé dans 1.7 mL d'éthanol jusqu'à dissolution complète du NaI. Le dérivé triiodophénol, préalablement solubilisé dans 0.7 mL d'éthanol est ensuite ajouté. Le milieu réactionnel est chauffé à reflux pendant 2 jours et demi, en s'assurant d'un débit d'eau dans le réfrigérant suffisamment élevé, de façon à observer une condensation rapide de l'éthanol.

L'avancement de la réaction est suivi par CCM (chromatographie sur couche mince) : Heptane/Acétate d'éthyle 5/5.

A la fin de la réaction, le milieu réactionnel est évaporé sous pression, puis on solubilise le solide obtenu dans une solution de NaOH (6M) de 6 mL. La phase aqueuse est ensuite lavée avec du DCM (dichlorométhane) (3 × 7 mL). Les phases organiques sont séchées sous $MgSO_4$. On obtient un solide jaunâtre. On procède à une purification sur colonne de silice, via un système de co-élution Heptane/acétate d'éthyle. A la suite de la purification, on obtient 0.133 mg d'un solide blanc.

Rendement molaire : 85 %

Pureté U.V. : 96 %

2. Estérification de l'alcool

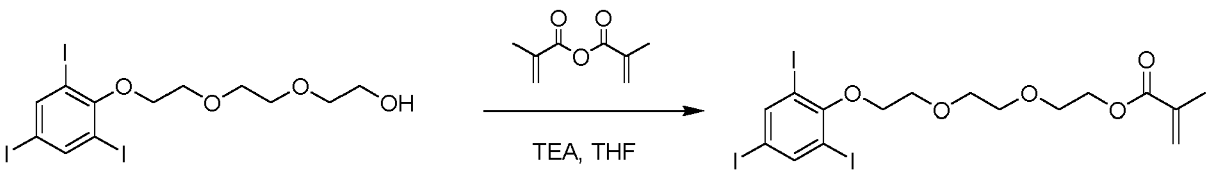


intermédiaire 1

Dans un tricol de 25 mL, équipé d'un réfrigérant, l'intermédiaire 1 (0.133 mg ; 0.22 mmol) est solubilisé dans 12 mL de THF anhydre. La TEA (triéthylamine) (0.10 mL ; 0.66 mmol) est ajoutée goutte à goutte. Le milieu réactionnel

est refroidi à une température inférieure à 5°C, puis l'anhydride méthacrylate (0.11 mL ; 0.66 mmol) est ajouté goutte à goutte sur une période de 5 minutes. Enfin, le milieu réactionnel est agité pendant une heure à une température inférieure à 5°C, puis à reflux pendant une nuit.

L'avancement de la réaction est suivi par CCM : Heptane/Acétate d'éthyle 5/5.

A la fin de la réaction, le milieu réactionnel est ramené à température ambiante, puis est suspendu dans 90 mL d'eau pendant une heure. On procède à un lavage avec du dichlorométhane (3 × 20 mL). Les phases organiques sont séchées sous $MgSO_4$, puis évaporées sous pression, jusqu'à l'obtention d'une huile orangée qui est ensuite purifiée sur colonne de silice, via un système de co-élution Heptane/Acétate d'éthyle. A la suite de la purification, on obtient 89.7 mg d'une huile transparente.

Rendement molaire : 74.5 %

Pureté U.V. : 96.6 %

3. Conclusion

La synthèse du MAETIP a été réalisée avec des rendements de synthèse d'environ 54% (comparable à celui de synthèse du MAOETIB), avec une pureté de la molécule finale d'environ 97%.

**Exemple 2 : Synthèse par polymérisation en suspension huile dans eau (phase directe) de microsphères selon l'invention à base de MAETIP, de taille 100-300 μm, 300-500 μm et 700-900 μm**

Les paramètres de synthèses sont adaptés à la taille de microsphères voulue (voir tableau 1)

a) Préparation de la phase aqueuse

Une solution aqueuse d'alcool polyvinylique (PVA) hydrolysé et de chlorure de sodium est préparée selon le protocole suivant :

i) Dissolution du PVA dans 5L d'eau apyrogène et agitation une nuit à 50°C.
ii) Ajout de NaCl et agitation à température ambiante pendant 4 heures.

b) Préparation de la phase organique

v) Pesée de chaque réactif et du toluène
w) Dissolution d'AIBN dans un volume de toluène
x) Dissolution dans un volume de toluène (dans un contenant différent) du poly(éthylène glycol) méthyl éther méthacrylate (m-$PEG_{300}$MA) (monomère hydrophile), du poly(éthylène glycol) diméthacrylate ($PEG_{1000}$DMA) (réticulant), de l'acide méthacrylique (AM ou MA) (monomère ionisable), du MAETIP (monomère radio-opaque) et du colorant puis ajout de l'hexanethiol (agent de transfert)
y) Ajout de la solution d'AIBN obtenue à l'étape w) à la solution de monomères de l'étape x)

c) Synthèse des microsphères selon l'invention

La solution aqueuse de PVA et de NaCl préparée à l'étape a) est versée dans un réacteur et chauffée à 50°C. La phase organique obtenue à l'étape b) est ensuite introduite dans le réacteur. Une agitation est appliquée avec un agitateur de type hélice pour obtenir des gouttelettes de phase dispersée du diamètre souhaité. La température est ensuite augmentée à 80°C et l'agitation est maintenue pendant 8 heures. Le mélange est ensuite filtré avec un tamis de 50 μm et les microsphères sont lavées à l'acétone, puis à l'éthanol puis à l'eau avant d'être tamisées avec des tamis de taille 50 μm, 100 μm, 300 μm et 500 μm, 700 μm, 900 μm et 1200 μm.

Le tableau 1 ci-dessous récapitule les paramètres principaux de la synthèse selon la taille des microsphères

**Tableau 1**

| Paramètres | 100-300 μm | 300-500 μm | 700-900 μm |
|---|---|---|---|
| Ratio O/W (huile/eau) | 1/11 | 1/8 | 1/6 |

(suite)

| Paramètres | 100-300 μm | 300-500 μm | 700-900 μm |
|---|---|---|---|
| Concentration massique de monomère dans la phase oragnique | 56 % | 56 % | 32 % |
| Vitesse d'agitation (TPM) | 230 | 180 | 120 |
| Phase aqueuse | 1% PVA (13-23 kDa) / 3% NaCl | 0.25% PVA (30-70 kDa) / 7% NaCl | 0.25% PVA (30-70 kDa) / 7% NaCl |
| Volume total (mL) | 240 | 240 | 240 |
| Volume de la phase aqueuse (mL) | 220 | 213 | 206 |
| n($PEG_{300}$MA)/n(phase monomère)* (%) | 64,98 | 64,98 | 54,98 |
| n($PEG_{1000}$DMA /n(phase monomère) * (%) | 5 | 5 | 5 |
| n(AM)/n(phase monomère)* | 10 | 10 | 10 |
| n(MAETIP)/n(phase monomère) * (%) | 20 | 20 | 30 |
| n(hexanethiol)/n($PEG_{300}$MA) (%) * | 3 | 3 | 3 |
| n(colorant)/n(phase monomère) * (%) | 0,02 | 0,02 | 0,02 |
| n(AIBN)/n(fonctions méthacrylates) * (%) | 1 | 1 | 1 |
| *n = nombre de moles | | | |

**Exemple 3 : Mesure du temps de suspension des MS selon l'invention et comparaison avec des MS comprenant un autre monomère radio-opaque que le MAETIP**

Le temps de suspension des MS selon l'invention synthétisées à l'exemple 1 dans un milieu d'injection (50/50 sérum physiologique/agent de contraste iodé) a été étudié et comparé à celui de MS témoins comprenant un monomère radio-opaque autre que le MAETIP..

Les MS témoins sont synthétisées selon le même protocole que celui de l'exemple 1 avec les mêmes constituants à l'exception du monomère radio-opaque. Les monomères radio-opaques suivants sont utilisés dans les MS témoins :

- Le MAOETIB de formule :

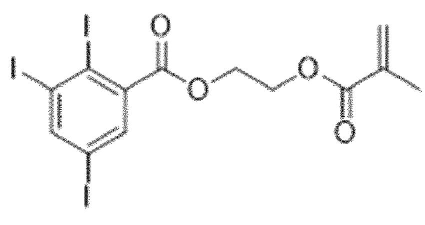

MAOETIB

- Le composé Vb décrit dans la demande WO 2021 /069528 de formule :

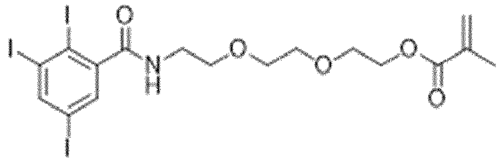

La mise en suspension a été réalisée en utilisant la méthode dite « Falcon » :.

Méthode Falcon

- 1 mL de microsphères radio-opaques est ajouté dans un tube Falcon® (ou Axygen®) dont le revêtement interne comprend du polypropylene (de polystyrène) de 15 mL, puis on complète le tube à « fleur d'eau » (observation d'un effet de bord, de façon à éjecter le plus d'air possible du tube) avec un mélange 50/50 d'eau distillée et d'agent de contraste ;
- On procède à la mise en suspension des microsphères, par des retournements successifs du tube pendant 3 minutes ;
- Le tube est mis au repos, et le temps de sédimentation (ou de crémage) des microsphères est mesuré.

**Résultats et discussion :**

a) Un premier essai de mise en suspension des microsphères a été réalisé dans des tubes Axygen® selon le protocole décrit précédemment. Les résultats obtenus sont les suivants :

**Tableau 2 : Temps de suspension des MS selon la méthode Falcon dans des tubes Axygen®**

| Monomère radio-opaque | Echantillon | Temps de suspension (en secondes) n = 3 | | | Moyenne |
|---|---|---|---|---|---|
| MAOETIB (témoin) | BF123 | 74 | 60 | 80 | 82 (± 12) |
| | BF124 | 88 | 94 | 99 | |
| | BF125 | 83 | 90 | 70 | |
| Composé Vb (témoin) | BF126 | 115 | 100 | 125 | 174 (± 47) |
| | BF127 | 205 | 205 | 220 | |
| | BF128 | 210 | 210 | 180 | |
| MAETIP (invention) | BF129 | 320 | 315 | 330 | 297 (± 36) |
| | BF130 | 323 | 318 | 305 | |
| | BF131 | 230 | 250 | 280 | |

A noter que dans chacun des cas, on observe une sédimentation des microsphères à la fin de la suspension dans le milieu d'injection.

b) Un second essai a été réalisé suivant les mêmes paramètres, dans un tube Falcon®. Les résultats obtenus sont les suivants :

**Tableau 3 : Temps de suspension des MS selon la méthode Falcon dans des tubes Falcon®**

| Monomère radio-opaque | Echantillon | Temps de suspension (en secondes) n = 3 | | | Moyenne |
|---|---|---|---|---|---|
| MAOETIB (témoin) | BF123 | 82 | 114 | 130 | 132 (± 26) |
| | BF124 | 108 | 150 | 160 | |
| | BF125 | 140 | 150 | 155 | |
| Composé Vb (témoin) | BF126 | 150 | 195 | 200 | 251 (± 94) |
| | BF127 | 240 | 360 | 440 | |
| | BF128 | 170 | 240 | 265 | |

(suite)

| Monomère radio-opaque | Echantillon | Temps de suspension (en secondes) n = 3 | | | Moyenne |
|---|---|---|---|---|---|
| MAETIP (invention) | BF129 | >600* | >600* | >600* | 571 (± 57) |
| | BF130 | >600* | >600* | >600* | |
| | BF131 | 470 | 470 | >600* | |

Dans chaque des cas, on observe une sédimentation des microsphères à la fin de la suspension dans le milieu d'injection.

Pour les deux essais, on observe que le temps de suspension des MS selon l'invention est bien supérieur à celui des MS témoins.

Les résultats annotés d'un « * » correspondent à un temps de suspension supérieur à 600 secondes. Il n'a pas été jugé pertinent de mesurer le temps de suspension au-delà de 600 secondes, les résultats obtenus par la suspension comprenant les MS selon l'invention étant largement supérieurs aux résultats observés pour les microsphères témoins.

## Revendications

**1.** Composé de formule (A) suivante :

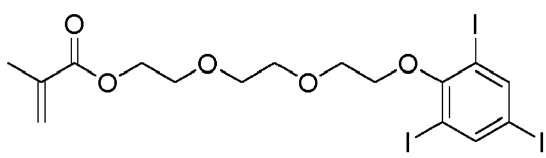
(A)

**2.** Utilisation du composé de formule (A) tel que défini dans la revendication 1 en tant que monomère halogéné radio-opaque.

**3.** Microsphères d'embolisation radio-opaques comprenant une matrice réticulée, ladite matrice étant à base d'au moins :

a) 20 % à 90 % de monomère hydrophile choisi parmi la N-vinylpyrrolidone et un monomère de formule (I) suivante :

$$(CH_2{=}CR_1)\text{-}CO\text{-}D \qquad (I)$$

dans laquelle :

- D représente O-Z ou NH-Z, Z représentant $(C_1\text{-}C_6)$alkyle, $-(CR_2R_3)_m\text{-}CH_3$, $-(CH_2\text{-}CH_2\text{-}O)_m\text{-}H$, $-(CH_2\text{-}CH_2\text{-}O)_m\text{-}CH_3$, $-C(R_4OH)_m$ ou $-(CH_2)_m\text{-}NR_5R_6$ avec m représentant un nombre entier de 1 à 30, de préférence m est égal à 4 ou 5
- $R_1$, $R_2$, $R_3$, $R_4$, $R_5$ et $R_6$ représentent, indépendamment les uns des autres, H ou un $(C_1\text{-}C_6)$alkyle ;

b) 5 % à 50 % de composé de formule (A) suivante :

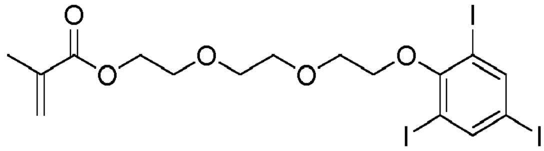
(A)

c) 1 % à 15 % de monomère réticulant hydrophile non biodégradable linéaire ou ramifié et présentant des groupes $(CH_2{=}(CR_{16}))$- à chacune de ses extrémités, chaque $R_{16}$ représentant indépendamment H ou un

($C_1$-$C_6$)alkyle ; et

d) 0,1 % à 10 % d'agent de transfert choisi parmi les halogénures d'alkyle et les thiols cycloaliphatiques ou aliphatiques ayant notamment de 2 à 24 atomes de carbone, et ayant éventuellement un autre groupe fonctionnel choisi parmi les groupes amino, hydroxy et carboxy,

les pourcentages des monomères a) à c) étant cités en mole par rapport au nombre de moles total de monomères et les pourcentages du composé d) étant cités en mole par rapport au nombre de moles du monomère hydrophile a).

**4.** Microsphères d'embolisation selon la revendication 3, dans lesquelles ladite matrice est à base du composé de formule générale (A) en une quantité supérieure à 7 % et inférieure ou égale à 50 %, avantageusement en une quantité supérieure à 10 % et inférieure ou égale à 50 %, avantageusement en une quantité supérieure à 15 % et inférieure ou égale à 50 %, plus avantageusement en une quantité supérieure à 15 % et inférieure ou égale à 35 %,et en particulier de 20 % à 30 % par mole, par rapport au nombre de moles total de monomères.

**5.** Microsphères d'embolisation selon la revendication 3 ou 4, dans lesquelles le monomère hydrophile a) est choisi dans le groupe constitué de N-vinylpyrrolidone, alcool vinylique, 2-hydroxyéthylméthacrylate, acrylate de sec-butyle, acrylate de n-butyle, acrylate de t-butyle, méthacrylate de t-butyle, méthylméthacrylate, N-diméthylaminoéthyl(méthyl)acrylate, N,N-diméthylaminopropyl-(méth)acrylate, *t*-butylaminoéthyl(méthyl)acrylate, N,N-diéthylaminoacrylate, (méth)acrylate de poly(éthylène oxyde), (méth)acrylate de méthoxy poly(éthylène oxyde), (méth)acrylate de butoxy poly(éthylène oxyde), (méth)acrylate de poly(éthylène glycol), (méth)acrylate de méthoxy poly(éthylène glycol), (méth)acrylate de butoxy poly(éthylène glycol), méthyl éther méthacrylate de poly(éthylène glycol) et leurs mélanges, avantageusement le monomère a) est du méthyl éther méthacrylate de poly(éthylène glycol).

**6.** Microsphères d'embolisation selon l'une quelconque des revendications 3 à 5, dans lesquelles le monomère réticulant hydrophile non biodégradable linéaire ou ramifié c) présente des groupes ($CH_2$=($CR_{16}$))CO- ou ($CH_2$=($CR_{16}$))CO-O- à ses au moins deux extrémités, chaque $R_{16}$ représentant indépendamment H ou un ($C_1$-$C_6$)alkyle.

**7.** Microsphères d'embolisation selon l'une quelconque des revendications 3 à 6, dans lesquelles l'agent de transfert d) est choisi parmi l'acide thioglycolique, le 2-mercaptoéthanol, le dodécanethiol, l'hexanethiol et leurs mélanges.

**8.** Microsphères d'embolisation selon l'une quelconque des revendications 3 à 7, dans lesquelles ladite matrice est en outre à base d'au moins un monomère ionisé ou ionisable de la formule (IV) suivante :

$$(CH_2=CR_{17})\text{-M-E} \qquad \text{(IV)}$$

dans laquelle

- $R_{17}$ représente H ou un ($C_1$-$C_6$) alkyle ;
- M représente une liaison simple ou un radical divalent ayant de 1 à 20 atomes de carbone ;
- E représente un groupe chargé ou ionisable ayant 100 atomes au plus, E étant avantageusement choisi dans le groupe constitué de -COOH, $-COO^-$, $-SO_3H$, $-SO_3^-$, $-PO_3H_2$, $-PO_3H^-$, $-PO_3^{2-}$, $-NR_{18}R_{19}$, $-NR_{20}R_{21}R_{22}^+$,
- $R_{18}$, $R_{19}$, $R_{20}$, $R_{21}$ et $R_{22}$ représentent indépendamment les uns des autres H ou un ($C_1$-$C_6$)alkyle.

**9.** Microsphères d'embolisation selon l'une quelconque des revendications 3 à 8, dans lesquelles ladite matrice est en outre à base d'au moins un monomère coloré de formule générale (VI) suivante :

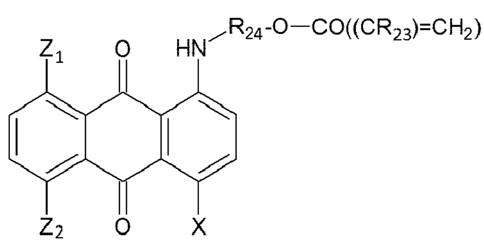

(VI),

dans laquelle,

- $Z_1$ et $Z_2$ représentent, indépendamment l'un de l'autre, H ou $OR_{25}$, $R_{25}$ représentant H ou un $(C_1-C_6)$alkyle, avantageusement $Z_1$ et $Z_2$ représentent H ;
- X représente H ou Cl, avantageusement H ;
- $R_{23}$ représente H ou un $(C_1-C_6)$alkyle, avantageusement un $(C_1-C_6)$alkyle, en particulier un méthyle ; et
- $R_{24}$ représente un groupe choisi parmi $(C_1-C_6)$alkylène linéaire ou ramifié, $(C_5-C_{36})$arylène, $(C_5-C_{36})$arylène-O-$R_{26}$, $(C_5-C_{36})$hétéroarytène et $(C_5-C_{36})$hétéroarylène-O-$R_{27}$, $R_{26}$ et $R_{27}$ représentant un $(C_1-C_6)$alkyle ou un $(C_1-C_6)$alkylène, avantageusement $R_{24}$ représente un groupe $-C_6H_4-O-(CH_2)_2-O$ ou $-C(CH_3)_2-CH_2-O$.

**10.** Microsphères d'embolisation selon l'une quelconque des revendications 3 à 9, dans lesquelles ladite matrice est en outre à base d'éléments visibles en imagerie par résonance magnétique (IRM) telles que des nanoparticules d'oxyde de fer, des chélates de gadolinium ou des chélates de magnésium, avantageusement des nanoparticules d'oxyde de fer.

**11.** Microsphères selon l'une quelconque des revendications 8 à 10, chargées d'une substance active avantageusement choisie dans le groupe constitué d'agents anti-inflammatoires, d'anesthésiques locaux, d'analgésiques, d'antibiotiques, d'agents anticancéreux, de stéroïde, d'antiseptique et d'un mélange de ceux-ci.

**12.** Microsphères d'embolisation selon l'une quelconque des revendications 8 à 10, chargé de macromolécules choisies dans le groupe constitué d'enzymes, d'anticorps, de cytokines, de facteurs de croissance, de facteurs de coagulation, d'hormones, de plasmides, d'oligonucléotides antisens, de siARN, de ribozymes, de DNA enzyme, d'aptamères, de protéines anti-inflammatoires, des protéines morphogènes osseuses (BMP), des facteurs pro-angiogéniques, des facteurs de croissance endothéliale vasculaire (VEGF) et des TGF-bêta, et des inhibiteurs de l'angiogenèse ou des anti-tyrosine kinases et leurs mélanges.

**13.** Composition pharmaceutique comprenant au moins une microsphère d'embolisation selon l'une quelconque des revendications 3 à 12, en association avec un véhicule pharmaceutiquement acceptable, avantageusement pour une administration par voie parentérale.

**14.** Kit comprenant une composition pharmaceutique telle que définie dans la revendication 13, en association avec un véhicule pharmaceutiquement acceptable pour une administration par voie parentérale, et au moins un moyen d'injection.

**15.** Kit comprenant d'une part une composition pharmaceutique telle que définie dans la revendication 13 et d'autre part au moins un agent de contraste pour l'imagerie par rayon X, par résonance magnétique ou par échographie, et éventuellement au moins un moyen d'injection pour une administration par voie parentérale, la composition pharmaceutique et l'au moins un agent de contraste étant conditionnés séparément.

Europäisches Patentamt
European Patent Office
Office européen des brevets

# RAPPORT DE RECHERCHE EUROPEENNE

Numéro de la demande
EP 22 30 5941

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | WO 2011/003902 A2 (BARTLING SOENKE [DE]; MARGEL SHLOMO [IL]; AVIV HAGIT [IL]) 13 janvier 2011 (2011-01-13) * exemples 1-6 * ----- | 1-15 | INV. A61K49/04 A61K49/00 A61K49/18 A61L24/04 C08F220/28 C08F226/10 A61K9/00 A61K31/00 A61K38/00 |
| A,D | WO 2021/069527 A1 (GUERBET SA [FR]) 15 avril 2021 (2021-04-15) * exemples 1-13 * * page 22, ligne 9 - page 23, ligne 14 * * revendications 1-15 * ----- | 1-15 | |
| A,D | WO 2021/069528 A1 (GUERBET SA [FR]) 15 avril 2021 (2021-04-15) * exemples 1-12 * * page 16, ligne 28 - page 19, ligne 2 * * revendications 1-17 * ----- | 1-15 | |
| A | JAYAKRISHNAN A ET AL: "Synthesis and polymerization of some iodine-containing monomers for biomedical applications", JOURNAL OF APPLIED POLYMER SCIENCE, JOHN WILEY & SONS, INC, US, vol. 44, no. 4, 5 février 1992 (1992-02-05), pages 743-748, XP009096023, ISSN: 0021-8995, DOI: 10.1002/APP.1992.070440421 * abrégé * * page 745, colonne 2, alinéa 1 * * page 746, colonne 2, alinéa 3 - page 748, colonne 2, alinéa 1 * ----- | 1-15 | DOMAINES TECHNIQUES RECHERCHES (IPC): A61K C08F A61L |

Le présent rapport a été établi pour toutes les revendications

1

| Lieu de la recherche | Date d'achèvement de la recherche | Examinateur |
|---|---|---|
| La Haye | 7 décembre 2022 | Monami, Amélie |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 22 30 5941

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

07-12-2022

| Document brevet cité au rapport de recherche | Date de publication | Membre(s) de la famille de brevet(s) | Date de publication |
|---|---|---|---|
| WO 2011003902 A2 | 13-01-2011 | EP 2451488 A2 | 16-05-2012 |
| | | US 2012184642 A1 | 19-07-2012 |
| | | WO 2011003902 A2 | 13-01-2011 |
| WO 2021069527 A1 | 15-04-2021 | CN 114555139 A | 27-05-2022 |
| | | EP 4041318 A1 | 17-08-2022 |
| | | KR 20220081358 A | 15-06-2022 |
| | | WO 2021069527 A1 | 15-04-2021 |
| WO 2021069528 A1 | 15-04-2021 | CN 114555659 A | 27-05-2022 |
| | | EP 4041319 A1 | 17-08-2022 |
| | | KR 20220079600 A | 13-06-2022 |
| | | WO 2021069528 A1 | 15-04-2021 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 2012120139 A
- WO 2012120138 A
- WO 2021069527 A
- WO 2021069528 A
- WO 2016166346 A
- WO 2016166339 A
- WO 2017005914 A
- WO 2017081178 A

### Littérature non-brevet citée dans la description

- **OSUGA et al.** *J. Vasc Interv Radiol.*, 2002, vol. 13, 929-34
- *CHEMICAL ABSTRACTS,* 10034-99-8
- *CHEMICAL ABSTRACTS,* 609-23-4
- *CHEMICAL ABSTRACTS,* 5197-62-6
- *CHEMICAL ABSTRACTS,* 7681-82-5
- *CHEMICAL ABSTRACTS,* 1310-73-2
- *CHEMICAL ABSTRACTS,* 760-93-0
- *CHEMICAL ABSTRACTS,* 121-44-8
- *CHEMICAL ABSTRACTS,* 64-17-5
- *CHEMICAL ABSTRACTS,* 141-78-6
- *CHEMICAL ABSTRACTS,* 142-82-5
- *CHEMICAL ABSTRACTS,* 75-09-2